# FASCICULE DE BREVET EUROPEEN

(11) **EP 0 627 916 B1**
(45) Date de publication et mention de la délivrance du brevet: **07.05.2003**
(21) Numéro de dépôt: 93905428.4
(22) Date de dépôt: 25.02.1993
(51) Int. Cl.: A61K 31/19, A61P 29/00

(54) **COMPOSITION PHARMACEUTIQUE UTILISABLE COMME ANALGESIQUE CONTENANT L'ACIDE (BENZOYL-3 PHENYL)-2 PROPIONIQUE-(R)**
ARZNEIMITTEL ENTHALTEND 2-(3-BENZOYLPHENYL)-PROPIONSÄURE ZUR VERWENDUNG ALS ANALGETIKUM
PHARMACEUTICAL COMPOSITION FOR USE AS A PAIN KILLER AND CONTAINING (BENZOYL-3 PHENYL)-2 PROPIONIC-(R) ACID

(30) Priorité: 28.02.1992 FR 9202336
(43) Date de publication de la demande: 14.12.1994
(73) Titulaire: Aventis Pharma S.A., 92160 Antony (FR)
(72) Inventeur: ABITEBOUL, Michel, F-92150 Suresnes (FR)
(74) Mandataire: Le Pennec, Magali
(86) Numéro de dépôt international: FR9300190
(87) Numéro de publication internationale: WO93016689

(56) Documents cités:
- US-A- 3 641 127
- J. PHARM. SCI. vol. 79, no. 5, 1990, pages 460 - 461 F. JAMALI 'Ketoprofen pharmacokinetics in humans: Evidence of enantiomeric inversion and lack of interaction.'
- J. PHARMACOL. EXP. THER. vol. 240, no. 2, 1987, page 637-641 A. ABAS 'Enantioselective disposition of 2-arylpropionic acid nonsteroidal anti-inflammatory drugs. IV. Ketoprofen disposition.'
- J. PHARM. PHARMACOL. vol. 35, no. 11, 1983, pages 693 - 704 A.J. HUTT 'The metabolic chiral inversion of 2-arylpropionic acids - a novel route with pharmacological consequences.'
- J. PHARM. PHARMACOL. vol. 37, no. 4, 1985, page 288 A.J. HUTT 'The metabolic chiral inversion of 2-arylpropionic acids - a novel route with pharmacological consequences - a correction.'
- AGENTS AND ACTIONS SUPPLEMENTS vol. 24, 1988, pages 76 - 84 K.M. WILLIAMS 'The contribution of enantiomers to variability in response to antiinflammatory drugs.'
- FUNDAM. CLIN. PHARMACOL. vol. 4, no. 6, 1990, pages 617 - 634 N. MULLER 'Pharmacological aspects of chiral nonsteroidal anti-inflammatory drugs.'
- EUR. J. CLIN. PHARMACOL. vol. 42, no. 3, Mars 1992, pages 237 - 256 A.M. EVANS 'Enantioselective pharmacodynamics and pharmacokinetics of chiral non-steroidal anti-inflamatory drugs.'
- J. CLIN. PHARMACOL. vol. 32, no. 10, Octobre 1992, pages 944 - 952 K. BRUNE 'Pure enantiomers of 2-arylpropionic acids: tools in pain research and improved drugs in rheumatology.'
- REYNOLDS, J.E.F. (ED.), 'MARTINDALE THE EXTRA PHARMACOPOEIA'. 29TH EDITION, 1989, THE PHARMACEUTICAL PRESS, LONDON.
- IKAWAGA SEIGO, SPAHN HILDEGARD, BENET L.Z., LIN E.T.: 'STEREOSELECTIVE BINDING OF THE GLUCURONIDE CONJUGATES OF CARPROFEN ENANTIOMERS TO HUMAN SERUM ALBUMIN' BIOCHEMICAL PHARMACOLOGY vol. 39, no. 5, 1990, pages 949 - 953
- KNADLER ET AL.: 'Plasma Protein Binding of Flurbiprofen: Enantioselectivity and influence of Pathophysiological Status' THE JOURNAL OF PHARMACOLOGY AND EXPERIMENTAL THERAPEUTICS vol. 249, no. 2, 1989, pages 378 - 385
- SIEBLER D., KINAVI A.: 'An Humanserumalbumin' ARZNEIM. FORSCH./DRUG RES. vol. 39, no. 1, 1989, pages 659 - 660
- RENDIC S., ALEBIC-KOLBAH T., KAIFEZ F., SUNJIC V. IL FARMACO ED. SC. vol. 35, no. 1, 2000,

## Description

La présente invention concerne des compositions pharmaceutiques utilisables comme analgésiques contenant l'acide (benzoyl-3 phényl)-2 propionique-(R) pratiquement exempt d'acide (benzoyl-3 phényl)-2 propionique-(S).

L'acide (benzoyl-3 phényl)-2 propionique (connu sous le nom de kétoprofène) sous forme racémique est un anti-inflammatoire non-stéroïdien particulièrement efficace qui a été décrit en particulier dans le brevet américain US 3,641,127.

Les études effectuées sur les produits connus pour leur activité anti-inflammatoire ont permis de montrer le rôle prépondérant des prostraglandines dans les mécanismes de l'inflammation. Les prostaglandines, qui sont produites par une enzyme, la cyclo-oxygénase, sont des médiateurs de l'inflammation. Par conséquent, en inhibant la production de la cyclo-oxygénase, la production des prostaglandines est diminuée ou anihilée ce qui conduit à une diminution ou à la disparition des manifestations inflammatoires. Ainsi la cyclo-oxygénase constitue la cible de choix des anti-inflammatoires non-stéroïdiens et les produits qui inhibent la cyclo-oxygénase doivent être potentiellement actifs sur la douleur, sur la fièvre et sur les manifestations inflammatoires.

Les acides aryl-2 propioniques, qui possèdent un atome de carbone asymétrique, sont constitués de deux isomères (R) et (S). Dans les essais pharmacologiques in vitro sur l'effet inhibiteur de cyclo-oxygénase, il a été montré que, dans cette famille de composés, l'isomère (S) est très nettement plus actif que l'isomère (R). Ainsi, pour le kétoprofène, l'effet inhibiteur est environ 50 fois plus élevé pour l'isomère (S) que pour l'isomère (R). Dans ces conditions, il peut être conclu que l'isomère (S) est le responsable de l'activité sur la douleur et l'inflammation.

Par ailleurs, les prostaglandines sont connues pour leur participation dans les mécanismes de protection gastrique en particulier par le biais de la synthèse du mucus. La diminution ou la disparition de la formation des prostaglandines contribue donc à la manifestation de la toxicité particulière des anti-inflammatoires non-stéroïdiens au niveau de l'estomac.

Les différences enregistrées entre l'activité in vitro et l'activité in vivo chez l'animal peuvent être expliquées par la conversion in vivo de l'isomère (R) en isomère (S). Cependant cette bioconversion n'est pas la même d'une espèce animale à l'autre et d'un produit à l'autre. Dans ces conditions, il est particulièrement difficile sinon impossible de pouvoir comparer l'activité des deux énantiomères chez l'animal afin d'avoir une orientation sur l'activité potentielle de chacun des isomères chez l'homme.

Alors que, pour la quasi totalité des acides aryl-2 propioniques anti-inflammatoires, la conversion chez l'homme de l'isomère (R) en isomère (S) se produit avec des cinétiques pouvant être variables, celle-ci ne se manifeste pas avec le kétoprofène-(R) ou avec le flurbiprofène-(R).

Dans le brevet américain US 4,868,214 ont été revendiquées l'utilisation du kétoprofène-(S) pour obtenir un effet analgésique rapide et une méthode de traitement de la douleur par administration de kétoprofène-(S). Cependant, les études cliniques qui ont été conduites sur le kétoprofène-(S) ont montré que le kétoprofène-(S) ne présentait pas de supériorité sur le kétoprofène racémique en ce qui concerne la rapidité d'action.

Des études comparatives ont été conduites avec le kétoprofène racémique et ses isomères (R) et (S) pour tenter d'expliquer l'absence d'une différence de comportement entre le kétoprofène racémique et le kétoprofène-(S)

Il a maintenant été trouvé, et c'est ce qui fait l'objet de la présente invention, que, d'une manière surprenante et inattendue, le kétoprofène-(R), qui manifeste une très faible activité in vitro sur l'inhibition de la cyclo-oxygénase, manifeste, chez l'homme, une activité analgésique remarquable, comparable à celle du kétoprofène-(S) associée à une toxicité gastro-entérique très nettement plus faible que celle du kétoprofène-(S).

En effet, contrairement aux conclusions conduisant à admettre que l'inhibition de la synthèse des prostaglandines contribuait à la manifestation d'effets analgésiques, il a maintenant été montré que l'effet analgésique d'un produit relevait d'un mécanisme différent et indépendant de celui attribuable à la synthèse des prostaglandines.

De plus, du fait de son absence ou de son faible effet inhibiteur de cyclo-oxygénase permettant ainsi la synthèse de prostaglandines favorisant la tolérance gastrique, l'isomère (R) du kétoprofène présente par rapport à l'isomère (S) une toxicité beaucoup plus faible ce qui constitue un avantage considérable au plan de la sécurité de l'emploi.

Ces résultats surprenants permettent l'utilisation du kétoprofène-(R) dans le traitement de la douleur et plus particulièrement de la douleur associée à une manifestation inflammatoire. Plus particulièrement, le kétoprofène-(R) est utile dans le traitement de la douleur du post-partum, de la douleur post-opératoire, de la douleur dentaire, des maux de tête, de la dysménorrhée, de la douleur musculo-squelettique, de la douleur tendino-ligamentaire ou de la douleur associée à des infections respiratoires.

La dose de kétoprofène-(R) nécessaire pour obtenir l'effet recherché dépend essentiellement de la nature de la douleur, de son intensité, de son origine et des facteurs propres au sujet à traiter et du mode d'administration. Chez l'homme, les doses seront comprises généralement entre 10 et 100 mg par jour pour un adulte et de préférence entre 15 et 75 mg. Cependant, dans des cas particuliers, la dose journalière pourra être substantiellement augmentée sous réserve que le médicament soit bien toléré par le malade.

La présente invention concerne les compositions contenant le kétoprofène-(R) éventuellement en association avec un ou plusieurs produits pharmaceutiquement acceptables et/ou physiologiquement actifs.

De préférence, les compositions sont celles couramment utilisées pour une administration orale. Cependant peuvent aussi être utilisées les compositions pour administration parentérale, rectale ou topique

Les compositions pour administration orale sont généralement des comprimés, des pilules, des granulés ou des poudres (notamment dans des capsules de gélatine ou des cachets). Dans ces compositions, le produit actif selon l'invention est mélangé à un ou plusieurs diluants inertes, tels que amidon, cellulose, saccharose, lactose ou silice. Ces compositions peuvent contenir également des substances autres que les diluants, par exemple des lubrifiants comme le stéarate de magnésium, ou le talc, des agents de désintégration tels que la méthylcellulose, la carboxyméthylcellulose, ou le laurylsulfate de sodium ou des colorants.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, le poplyéthylèneglycol, les huiles végétales; en particulier l'huile d'olive, des esters organiques injectables tels que l'oléate d'éthyle ou d'autres solvants organiques convenables. Ces compositions peuvent également contenir des adjuvants, en particulier des agents mouillants, isotonisants, émulsifiants, dispersants ou stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions stériles solides qui peuvent être dissoutes au moment de l'emploi dans de l'eau stérile ou tout autre milieu stérile injectable.

Les compositions pour administration rectale sont les suppositoires ou les capsules rectales qui contiennent outre le principe actif des excipients tels que le beurre de cacao, les glycérides semi-synthétiques ou des polyéthylèneglycols.

Les compositions pour administration topique peuvent être par exemple des crèmes, des pommades, des poultices, des lotions, des collyres, des collutoires, des gouttes nasales ou des aérosols.

Dans les compositions pour administration orale, parentérale, rectale ou topique, le kétoprofène-(R) peut être sous forme d'acide ou sous forme de sel pharmaceutiquement acceptable. Comme sels pharmaceutiquement acceptables peuvent être cités les sels avec les bases minérales ou organiques ou avec les acides aminés basiques tels que la lysine ou l'arginine.

Le kétoprofène-(R) utilisé dans les compositions selon l'invention peut être obtenu par toute méthode décrite dans la littérature pour la préparation des isomères optiques des acides aryl-2 propioniques telles que la séparation des constituants du mélange racémique par chromatographie sur une colonne chirale ou par dédoublement d'un sel optiquement actif, par synthèse chimique ou par hydrolyse chimique ou enzymatique d'un ester ou de l'amide correspondant.

Plus particulièrement, le kétoprofène-(R) peut être obtenu dans les conditions décrites dans le brevet américain US 3,641,127.

Plus particulièrement encore, le kétoprofène-(R) peut être obtenu par dédoublement d'un sel du kétoprofène racémique avec une base organique optiquement active telle que la cinchonidine, la cinchonine ou la phényléthylamine dans les conditions décrites dans la demande DE 3 824 353.

Plus particulièrement encore le kétoprofène-(R) peut être obtenu par hydrolyse enzymatique de l'amide du kétoprofène au moyen d'une enzyme stéréosélective en utilisant, par exemple, le procédé décrit dans le brevet européen EP 0 330 529. Selon ce brevet, l'amide racémique du kétoprofène est hydrolysé stéréosélectivement, en présence d'un microorganisme choisi parmi Brevibacterium R 312 (CBS 717-73), Corynebacterium N 771 (FERM P 4445) et Corynebacterium N 774 (FERM P 4446) ou d'une enzyme issue de ce microorganisme, en kétoprofène-(S) qui est séparé de l'amide du kétoprofène-(R) qui peut être hydrolysé en kétoprofène-(R) selon les méthodes connues permettant d'hydrolyser un amide en acide dans des conditions non racémisantes. Généralement, l'hydrolyse de l'amide du kétoprofène-(R) peut être effectuée à une température inférieure à 50°C en milieu acide (acide chlorhydrique) éventuellement en présence d'un catalyseur ou bien par action de N₂O₄ dans un solvant organique tel que l'acide acétique.

L'exemple suivant illustre une composition selon la présente invention.

### EXEMPLE

1) On prépare le kétoprofène-(R) par hydrolyse, dans des conditions non racémisantes, de l'amide correspondant obtenu après séparation du kétoprofène-(S) dans les conditions décrites dans le brevet EP 0 330 529
2) On prépare selon la technique habituelle des comprimés dosés à 25 mg de kétoprofène-(R) ayant la composition suivante :
- kétoprofène-(R) 25 mg
- amidon 60 mg
- lactose 50 mg
- stéarate de magnésium 2 mg

3) Les effets bénéfiques du kétoprofène-(R) sont mis en évidence en réalisant, avec une composition selon l'invention en comparaison avec le kétoprofène-(S) et le kétoprofène racémique dans les mêmes conditions, les essais cliniques suivants :
1) tolérance gastrique :
On administre, en double aveugle, à des volontaires sains (24) une dose unique de kétoprofène racémique (50 mg), de kétoprofène-(R) (25 mg) et kétoprofène-(S) (25 mg). Deux heures après l'administration, une fibroscopie gastrique permet de mesurer un indice de toxicité en utilisant le score de Lanza [entre 0 (normal) et 4 (ulcère étendu)].
Les résultats sont rassemblés dans le tableau suivant :

| SCORE | 0 | 1 | 2 | 3 | 4 |
|---|---|---|---|---|---|
| Kètoprofène-(R) 25 mg | 21 | 2 | 1 | 0 | 0 |
| Kétoprofène-(S) 25 mg | 19 | 1 | 4 | 0 | 1 |
| Kétoprofène-(R,S) 50 mg | 20 | 0 | 4 | 0 | 0 |

Les résultats montrent que le kétoprofène-(R) à la dose de 25 mg donne moins de lésions gastriques sévères (scores 2, 3 et 4) que le kétoprofène-(S) à la dose de 25 mg et le kétoprofène racémique à la dose de 50 mg.
2) effet analgésique :
On administre, en double aveugle, à des malades (190) ayant subi une extraction dentaire sous anesthésie, une dose unique de kétoprofène-(R) (25 mg), de kétoprofène-(S) (25 mg et 12,5 mg) et de kétoprofène racémique (50 mg).
La douleur est évaluée par échelle visuelle et verbale.

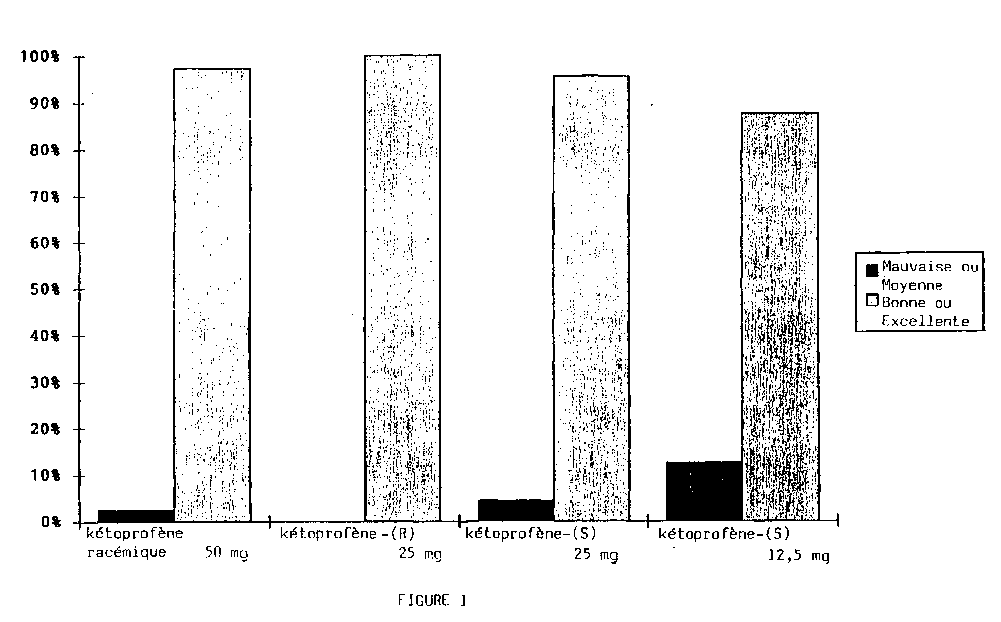

La figure 1 donne les résultats de la tolérance par malade sur une population randomisée

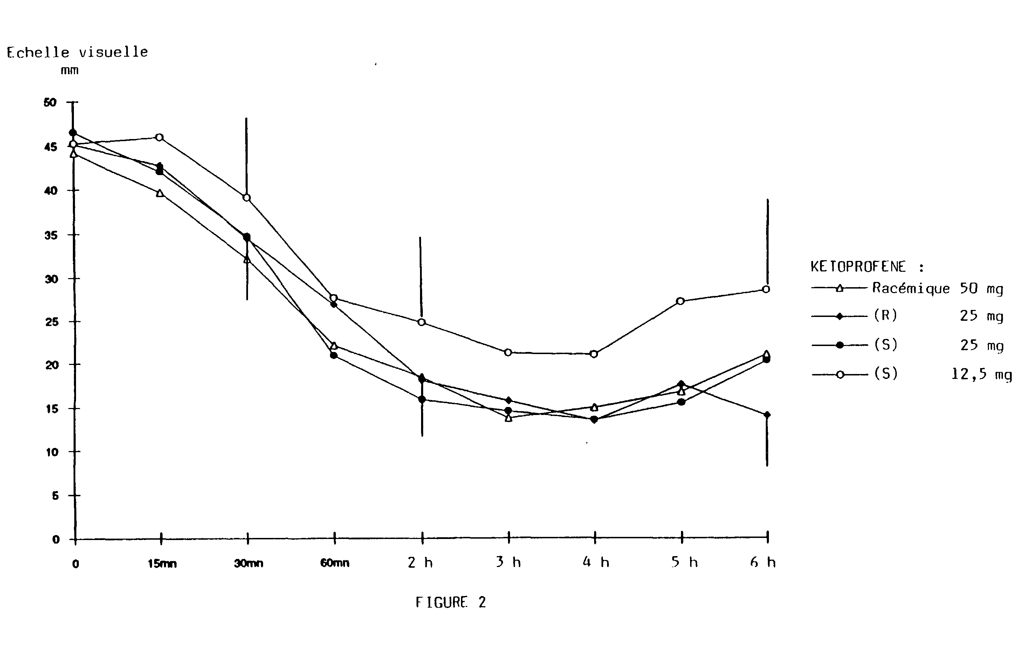

La figure 2 représente les résultats obtenus sur la population moyenne estimable en fonction du temps en utilisant une échelle visuelle analogique.
Les résultats montrent que le kétoprofène-(R) à la dose de 25 mg est aussi efficace sur la douleur que le kétoprofène-(S) à la dose de 25 mg et que le kétoprofène racémique à la dose de 50 mg et que ces trois produits [racémique et isomères (R) et (S)] sont plus actifs que le kétoprofène-(S) à la dose de 12,5 mg.

## Revendications

1. Utilisation de l'acide (benzoyl-3 phényl)-2 propionique-(R) pratiquement exempt d'acide (benzoyl-3 phényl)-2 propionique-(S), ou de ses sels pharmaceutiquement acceptables pour la préparation d'une composition pharmaceutique utilisable comme analgésique.

2. Utilisation de l'acide (benzoyl-3 phényl)-2 propionique-(R) pratiquement exempt d'acide (benzoyl-3 phényl)-2 propionique-(S) ou de ses sels pharmaceutiquement acceptables selon la revendication 1 pour la préparation d'une composition pharmaceutique utilisable dans le traitement de la douleur.

3. Utilisation de l'acide (benzoyl-3 phényl)-2 propionique-(R) pratiquement exempt d'acide (benzoyl-3 phényl)-2 propionique-(S) ou de ses sels pharmaceutiquement acceptables selon l'une quelconque des revendications précédentes pour la préparation d'une composition pharmaceutique utilisable dans le traitement des douleurs du post-partum, des douleurs post-opératoires, des douleurs dentaires, des maux de tête, de la dysménorrhée, des douleurs musculo-squelettiques, des douleurs tendino-ligamentaires ou des douleurs associées à des infections respiratoires.

4. Utilisation pour la préparation d'une composition pharmaceutique selon l'une quelconque des revendications précédentes pour administration de 10 à 100 mg/jour chez un adulte d' acide (benzoyl-3 phényl)-2 propionique-(R).

5. Utilisation pour la préparation d'une composition pharmaceutique selon l'une quelconque des revendications précédentes pour administration par voie orale.

6. Utilisation pour la préparation d'une composition pharmaceutique selon l'une quelconque des revendications précédentes pour administration sous forme de comprimés, de granulés, de capsules ou de poudres.

7. Utilisation pour la préparation d'une composition pharmaceutique selon l'une quelconque des revendications 1 à 4 pour administration sous forme de suppositoires.

8. Utilisation pour la préparation d'une composition pharmaceutique selon l'une quelconque des revendications précédentes **caractérisée en ce que** l'acide (benzoyl-3 phényl)-2 propionique-(R) est sous forme d'un sel d'addition avec une base minérale ou azotée ou avec un acide aminé basique.

9. Utilisation pour la préparation d'une composition pharmaceutique selon la revendication précédente 8 **caractérisée en ce que** l'acide aminé basique est choisi parmi la lysine et l'arginine.

10. Utilisation pour la préparation d'une composition pharmaceutique selon l'une quelconque des revendications précédentes **caractérisée en ce que** la toxicité gastro-entérique associée est très nettement plus faible que celle du kétoprofène-(S).

11. Procédé de préparation d'un médicament pour traiter la douleur **caractérisé en ce qu'**on met en oeuvre l'acide (benzoyl-3 phényl)-2 propionique-(R) en tant que constituant essentiel dudit médicament.

## Patentansprüche

1. Verwendung von (R)-2-(3-Benzoylphenyl)-propionsäure, die praktisch frei von (S)-2-(3-Benzoylphenyl)propionsäure ist, oder ihrer pharmazeutisch annehmbaren Salze zur Herstellung einer pharmazeutischen Zusammensetzung, die als Analgetikum verwendbar ist.

2. Verwendung von (R)-2-(3-Benzoylphenyl)-propionsäure, die praktisch frei von (S)-2-(3-Benzoylphenyl)propionsäure ist, oder ihrer pharmazeutisch annehmbaren Salze gemäß Anspruch 1 zur Herstellung einer pharmazeutischen Zusammensetzung, die bei der Schmerzbehandlung verwendbar ist.

3. Verwendung von (R)-2-(3-Benzoylphenyl)-propionsäure, die praktisch frei von (S)-2-(3-Benzoylphenyl)propionsäure ist, oder ihrer pharmazeutisch annehmbaren Salze gemäß einem der vorhergehenden Ansprüche zur Herstellung einer pharmazeutischen Zusammensetzung, die bei der Behandlung der Schmerzen nach der Geburt, der postoperativen Schmerzen, der Zahnschmerzen, der Kopfschmerzen, der Dysmenorrhoe, der Muskel- und Skelettschmerzen, der Sehnenund Bänderschmerzen oder der Schmerzen, die mit Atemwegsinfektionen verbunden sind, verwendbar ist.

4. Verwendung zur Herstellung einer pharmazeutischen Zusammensetzung gemäß einem der vorhergehenden Ansprüche zur Verabreichung von 10 bis 100 mg/Tag (R)-2-(3-Benzoylphenyl)-propionsäure bei einem Erwachsenen.

5. Verwendung zur Herstellung einer pharmazeutischen Zusammensetzung gemäß einem der vorhergehenden Ansprüche zur oralen Verabreichung.

6. Verwendung zur Herstellung einer pharmazeutischen Zusammensetzung gemäß einem der vorhergehenden Ansprüche zur Verabreichung in Form von Tabletten, Granulat, Kapseln oder Pulvern.

7. Verwendung zur Herstellung einer pharmazeutischen Zusammensetzung gemäß einem der Ansprüche 1 bis 4 zur Verabreichung in Form von Zäpfchen.

8. Verwendung zur Herstellung einer pharmazeutischen Zusammensetzung gemäß einem der vorhergehenden Ansprüche, **dadurch gekennzeichnet, dass** die (R)-2-(3-Benzoylphenyl)-propionsäure in Form eines Additionssalzes mit einer mineralischen oder stickstoffhaltigen Base oder mit einer basischen Aminosäure vorliegt.

9. Verwendung zur Herstellung einer pharmazeutischen Zusammensetzung gemäß dem vorhergehenden Anspruch 8, **dadurch gekennzeichnet, dass** die basische Aminosäure unter Lysin und Arginin ausgewählt ist.

10. Verwendung zur Herstellung einer pharmazeutischen Zusammensetzung gemäß einem der vorhergehenden Ansprüche, **dadurch gekennzeichnet, dass** die damit verbundene gastroenterische Toxizität deutlich geringer als die von (S)-Ketoprofen ist.

11. Verfahren zur Herstellung eines Medikaments zur Schmerzbehandlung, **dadurch gekennzeichnet, dass** man (R)-2-(3-Benzoylphenyl)-propionsäure als wesentlichen Bestandteil besagten Medikaments einsetzt.

## Claims

1. Use of (R)-(3-benzoylphenyl)-2-propionic acid which is practically free of (S)-(3-benzoylphenyl)-2-propionic acid, or of its pharmaceutically acceptable salts for the preparation of a pharmaceutical composition for use as a painkiller.

2. Use of (R)-(3-benzoylphenyl)-2-propionic acid which is practically free of (S)-(3-benzoylphenyl)-2-propionic acid, or of its pharmaceutically acceptable salts according to Claim 1 for the preparation of a pharmaceutical composition for use in the treatment of pain.

3. Use of (R)-(3-benzoylphenyl)-2-propionic acid which is practically free of (S)-(3-benzoylphenyl)-2-propionic acid, or of its pharmaceutically acceptable salts according to any one of the preceding claims, for the preparation of a pharmaceutical composition for use in the treatment of postpartum pain, of postoperative pain, of dental pain, of headaches, of dysmenorrhoea, of musculoskeletal pain, tendon-ligament pain or pain associated with respiratory infections.

4. Use, for the preparation of a pharmaceutical composition according to any one of the preceding claims, for the administration of 10 to 100 mg/day, in an adult, of (R)-(3-benzoylphenyl)-2-propionic acid.

5. Use for the preparation of a pharmaceutical composition according to any one of the preceding claims for administration by the oral route.

6. Use for the preparation of a pharmaceutical composition according to any one of the preceding claims for administration in the form of tablets, granules, capsules or powders.

7. Use for the preparation of a pharmaceutical composition according to any one of Claims 1 to 4 for administration in the form of suppositories.

8. Use for the preparation of a pharmaceutical composition according to any one of the preceding claims, **characterized in that** the (R)-(3-benzoylphenyl)-2-propionic acid is in the form of an addition salt with an inorganic or a nitrogenous base or with a basic amino acid.

9. Use for the preparation of a pharmaceutical composition according to the preceding Claim 8, **characterized in that** the basic amino acid is chosen from lysine and arginine.

10. Use for the preparation of a pharmaceutical composition according to any one of the preceding claims, **characterized in that** the associated gastroenteric toxicity is very markedly lower than that of (S)-ketoprofen.

11. Method of preparing a medicament for treating pain, **characterized in that** (R)-(3-benzoylphenyl)-2-propionic acid is used as the main constituent of the said medicament.
